(19) Europäisches Patentamt
European Patent Office
Office européen des brevets

(11) Veröffentlichungsnummer: **0 147 461 B1**

(12) **EUROPÄISCHE PATENTSCHRIFT**

(45) Veröffentlichungstag der Patentschrift: **08.01.92**

(51) Int. Cl.[5]: **C07C 405/00**, C07D 317/20, C07D 319/06, C07D 263/14, C07D 271/06, C07D 493/08

(21) Anmeldenummer: **84902782.6**

(22) Anmeldetag: **05.07.84**

(86) Internationale Anmeldenummer: **PCT/DE84/00139**

(87) Internationale Veröffentlichungsnummer: **WO 85/00367 (31.01.85 85/03)**

# (54) 11-HALOGEN-PROSTANDERIVATE, VERFAHREN ZU IHRER HERSTELLUNG UND IHRE ANWENDUNG ALS ARZNEIMITTEL.

(30) Priorität: **08.07.83 DE 3325175**

(43) Veröffentlichungstag der Anmeldung: **10.07.85 Patentblatt 85/28**

(45) Bekanntmachung des Hinweises auf die Patenterteilung: **08.01.92 Patentblatt 92/02**

(84) Benannte Vertragsstaaten: **AT BE CH DE FR GB LI LU NL SE**

(56) Entgegenhaltungen:

**Prostaglandins, Band 16, Nr. 1, Juli 1978, Los Altos, Cal., US; C.E. ARRONIZ et al.: "Synthesis of Ring Halogenated Prostaglandins (1)", Seiten 47-60, siehe Seiten 47, 52-59 (In der Anmeldung erwähnt)**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **VORBRÜGGEN, Helmut**
**Wilkestr. 7**
**W-1000 Berlin 27(DE)**
Erfinder: **SCHWARZ, Norbert**
**Stubenrauchstr. 31**
**W-1000 Berlin 41(DE)**
Erfinder: **LOGE, Olaf**
**Bekassinenweg 37**
**W-1000 Berlin 27(DE)**
Erfinder: **STÜRZEBECHER, Claus-Steffen**
**Kuckucksweg 6**
**W-1000 Berlin 33(DE)**
Erfinder: **ELGER, Walter**
**Schorlemer Allee 12b**
**W-1000 Berlin 33(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Chemistry, Biochemistry, and Pharmacological Activity of Prostanoids, herausgegeben von S.M. Roberts und F. Scheinmann, Oxford, GB; J.M. MUCHOWSKI: "Synthesis and Bronchial Dilator Activity of Some Novel Prostaglandin Analogues (1)", Seiten 39-60, siehe Seiten 54-57

Chemical Abstracts, Band 92, Nr. 5, 4. Februar 1980, Columbus, Ohio, US; V. V: BEZUGLOV et al: "Synthesis of fluoroprostaglandins. I.11-Fluoro and 15-fluoro prostaglandins", siehe S. 734, Zusammenfassung Nr. 41402a, Bioorg. Khim., 1979, 5(10), 1531-6 (Russ)

## Beschreibung

Prostaglandin $D_2$ ist ein natürliches Prostaglandin, das in vielen Organen, vor allem aber im Gehirn vorkommt und eine Vielzahl von biologischen wirkungen ausübt. Prostaglandin $D_2$ wie auch Analoga des $PGD_2$ (vergl. DOS 25 17 773) sind aber chemisch noch weniger stabil als die Prostaglandine der E-Reihe.

Daher war es von Interesse, chemisch stabile Analoga der Prostaglandin D-Reihe mit gleicher oder ähnlicher biologischer Aktivität wie $PGD_2$ zu entwickeln. In der chemischen Literatur sind einige chemisch stabile 11α- bsw. 11β-Fluor- Oder-Chlor-Derivate der 9α,15α-Dihydroxy-5-cis-13-trans-prostadiensaure beschrieben worden [E. Arroniz et al, Prostaglandins 16, 47 (1978)], aber abgesehen von relativ schwachen bronchodilatorischen Eigenschaften des 11α-Fluorderivates wurden in dieser Arbeit keine biologischen Daten diskutiert bzw. keine weiteren 11-Halogenprostansäureanaloga synthetisiert. J.M. Muchowski beschreibt in Chemistry, Biochemistry and Pharmacological Activity of Prostanoids (erschienen in Pergamon Press und herausgegeben von S.M. Roberts et al., Seite 39-60, Oxford) eine Reihe von 11-Fluor- und 11-Chlor-5-cis-13-trans-prostadiensäuren, die alle in der unteren Seitenkette in 15-Stellung einen Pentylrest tragen und deren bronchodilatorische Wirkung mit $PGE_2$ vergleichbar oder kleiner als die von $PGE_2$ ist. In Chemical Abstract 92: 41402a (1980) wird die Herstellung von 11-Fluor-9α.15α-dihydroxy-11-desoxy-5-cis-13-trans-prostadiensäuremethylestergenannt. In den EP 030377, 069696 und 081455 werden 9-Halogenprostaglandine unter Schutz gestellt.

Wir haben nun gefunden, daß 11-Fluor-, 11-Chlor- und 11-Brom-derivate der 9α, 15α-Dihydroxy-prostansäure sowie insbesondere modifizierte und dadurch metabolisch stabile Analoga dieser 11-Halogenprostansäuren überraschenderweise interessante biologische Wirkungen besitzen, wobei die Strukturveränderungen das Ziel haben, die Wirkungsdauer sowie die Selektivität der biologischen Wirkungen zu steigern.

Die Erfindung betrifft 11-Halogen-prostanderivate der allgemeinen Formel I

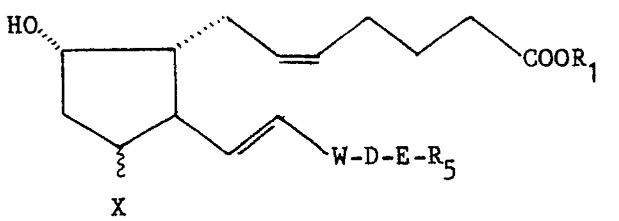

(I),

worin

- X F, Cl oder Br,
- $R_1$ Methyl wenn X, F oder Cl, D die Gruppen $-CH_2-$ oder $-CH(CH_3)-CH_2-$,E Sauerstoff oder eine direkte Bindung und $R_5$ Phenyl oder den Rest $CH=C(CH_3)$ 2 bedeuten, oder Wasserstoff,
- W eine Äthylendioxy-methylengruppe, wenn $R_1$ Methyl, X Cℓ, D die Gruppe $-CH_2-$, E Sauerstoff und $R_5$ Phenyl bedeuten oder eine Hydroxymethylengruppe,
- D die Gruppe $-CH_2-$, wenn E Sauerstoff und $R_5$ Phenyl bedeuten, oder D die Gruppe $-C(CH_3)_2-CH_2-$, wenn $ER_5$ n-Propyl oder den Rest $CH=C(CH_3)_2$ bedeuten,
- $DER_5$ den Rest $-CH(CH_3)-CH_2-CH=C(CH_3)_2$,
- $R_5$ $C_3$-$C_6$ Cycloalkyl, wenn D und E gemeinsam eine direkte Bindung bedeuten, oder Phenyl, wenn D eine $-CH_2$-Gruppe und E Sauerstoff bedeuten.

Die Halogenatome in 11-Position der Formel I können sowohl α- als auch β-ständig sein.

Als Cycloalkylgruppe $R_5$ kommen in Betracht: Cyclopropyl, Cyclobutyl, Cyclopentyl, Cyclohexyl.

Die Erfindung betrifft außerdem Verfahren zur Herstellung von 11-Halogen-prostanderivaten der allgemeinen Formel I, dadurch gekennzeichnet, daß man in an sich bekannter Weise eine Verbindung der allgemeinen Formel II

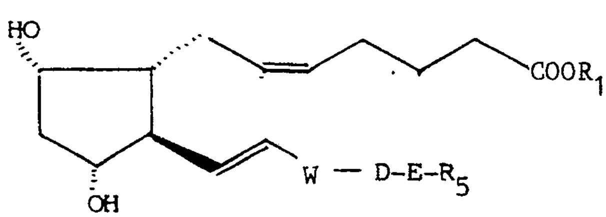

(II),

worin
W, D, E, $R_1$ und $R_5$ die oben angegebenen Bedeutungen haben und freie OH-Gruppen in 9- und 15-Stellung gegebenenfalls geschützt sind

a) mit Triphenylphosphin/$CCl_4$ oder $C_2Cl_6$ direkt in die entsprechenden 11β-Chlorderivate überführt.

b) durch Reaktion mit p-Toluolsulfonylchlorid oder -anhydrid und anschließende Umsetzung mit einem Fluorid oder Chlorid zu den den entsprechenden 11β-Fluor- oder -Chlor-verbindungen umsetzt oder

c) mit Diäthylazodicarboxylat/Triphenylphosphin/Zink-tosylat in Tetrahydrofuran in die entsprechenden 11β-Tosyloxyderivate überführt und diese mit Fluorid- oder Chloridsalzen zu den entsprechenden 11α-Halogenprostansäurederivaten umsetzt und gegebenfalls anschließend die vorhandenen Schutzgruppen abspaltet.

Die Umsetzung von II mit Triphenylphosphin/$CCl_4$ oder $C_2Cl_6$ erfolgt in an sich bekannter Weise, zum Beispiel in absolutem Acetonitril oder Pyridin als Lösungsmittel. Wegen des großen Raumbedarfs der dabei entstehenden 0-Triphenylphosphoniumsalze reagiert bei 9α,11α-Diolen selektiv zuerst die weniger gehinderte 11α-Hydroxylgruppe. Ein selektiver Schutz der 15α-Hydroxylgruppe gelingt durch Acylierung der Phenylborsäureester der 9α,11α-Hydroxylgruppen in 9α,11a,15α-Trihydroxyprostansäureestern.

Befinden sich Raum-beanspruchende Substituenten wie Methylgruppen in 15- oder insbesondere in 16-Stellung, so ist auch ein Schutz der 15-Hydroxylgruppe überflüssig, da selektiv zuerst nur die 11α-Hydroxylgruppe mit Triphenylphosphin/$CCl_4$ bzw. $C_2Cl_6$ reagiert. Dies gilt mit gewisser Einschränkung auch für die Umsetzung von Verbindungen der allgemeinen Formel II mit Triphenylphosphin / Azoester / Zn-Tosylat sowie mit aktivierten Sulfosäurederivaten. Diese Reaktionen werden in an sich bekannter Weise mit einem gegebenenfalls halogenierten Alkyl- oder Arylsulfosäurechlorid oder -anhydrid in Gegenwart eines Amins, wie beispielsweise Pyridin, 4-Dimethylaminopyridin oder Triäthylamin bei Temperaturen zwischen -20 °C und +100 °C durchgeführt. Die nucleophile Substitution der 11α- oder 11β-Sulfonate mit einem ionischen Halogenid, wie vorzugsweise Tetrabutylammoniumfluorid, CsF, Tetrabutylammoniumchlorid oder LiCl verläuft in einem inerten Lösungsmittel, wie beispielsweise Dimethylformamid, Acetonitril, Hexamethylphosphorsäuretriamid, Tetrahydrofuran, bei Temperaturen zwischen 0 °C und 80 °C.

Für die Synthese von 9α-Acyloxy- bzw. 9α,15α-Di-Acyloxyderivaten, in denen eine oder beide Hydroxylgruppen selektiv geschützt sind, wird zweckmäßigerweise zuerst ausgehend von einem 11α-Tetrahydropyranyl-13-tert-butyldimethyl-silyläther-Corey-lacton nach DOS 31 07 100 mittels DIBAH-Reduktion, Wittig-Reduktion, $CH_2N_2$-Veresterung und 0-Benzoylierung der 9-Hydroxylgruppe die obere Kette synthetisiert. Abspaltung der Silylgruppe mit einem Fluorid, Oxydation des 13-Alkohols zum 13-Aldehyd sowie Wittig-Reaktion mit substituierten Horner-Wittig-Reagenzien führen dann zur Konstruktion der unteren Kette. Schließlich ergibt die Reduktion der 15-Ketogruppe mit $NaBH_4$ und anschließende Epimeren-Trennung den 9α-benzoylierten 11α-letrahydropyranyl(THP)-15α-alkohol.

Im Falle von Substituenten in 16-Stellung, wie zum Beispiel der 16,16-Dimethylanaloga, wird nun die 11α-THP-Gruppe mit Essigsäure-$H_2O$-THF abgespalten und der 9α-Benzoyloxy-11α,15α-dihydroxy-16,16-dimethylprostansäuremethylester direkt mit Triphenylphosphin-Azoester-Zinktosylat umgesetzt zum 9α-Benzoyloxy-11β-tosyloxy-15α-hydroxy-16,16-dimethylprostansäuremethylester.

Falls die 15α-Hydroxylgruppe relativ ungehindert ist, muß diese Hydroxylgruppe ebenfalls benzoyliert werden zum 9α,15α-Di-benzoyloxy-11α-THP-prostansäuremethylester, bevor selektiv die 11α-THP-Gruppe abgespalten und dann zu den entsprechenden 11β-Halogen- oder 11β-Tosyloxyderivaten umgesetzt werden kann.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wäßrigen Lösung einer organischen Säure, wie zum Beispiel Oxalsäure, Essigsäure, Propionsäure u.a., oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Verseifung der Acylgruppen erfolgt beispielsweise mit Alkali- oder Erdalkalicarbonaten oder -hydroxyden in einem Alkohol oder in der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt. Bevorzugt sind die Kaliumsalze.

Als Erdalkalicarbonate und -hydroxyde sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe

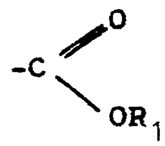

bei welcher $R_1$ eine Methylgruppe darstellt, erfolgt nach den dem Fachmann bekannten Methoden. Die 1-Carboxy-Verbindungen werden beispielsweise mit Diazomethan in an sich bekannter Weise umgesetzt. Die Veresterung mit Diazokohlenwasserstoffen erfolgt zum Beispiel dadurch, daß man eine Lösung des Diazokohlenwasserstoffs in einem inerten Lösungsmittel, vorzugsweise in Diäthyläther, mit der 2-Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Prostaglandinderivate der allgemeinen Formel I mit $R_1$ in der Bedeutung eines Wasserstoffatoms können mit geeigneten Mengen der entsprechenden anorganischen Basen unter Neutralisierung in ein Salz überführt werden. Beispielsweise erhält man beim Lösen der entsprechenden PG-Säuren in Wasser, das die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Diäthyläther, Acetonitril oder Benzol, gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Enthält das Ausgangsprodukt OH-Gruppen im Prostanrest, so können auch diese OH-Gruppen reagieren, so daß man in diesen Fällen zweckmäßigerweise von Ausgangsprodukten ausgeht, in denen diese durch vorzugsweise leicht abspaltbare Äther- oder Acylreste intermediär geschützt sind.

Die als Ausgangsmaterial dienenden Verbindungen der allgemeinen Formel II mit einer geschützten Hydroxylgruppe in 15-Stellung und einer freien Hydroxylgruppe in 9-Stellung

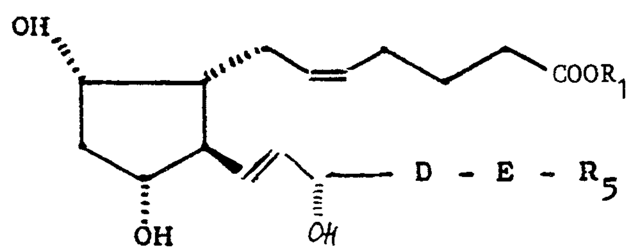

(IV)

können beispielsweise hergestellt werden, indem man $9\alpha,11\alpha,15\alpha$-Trihydroxyprostansäureester IV mit Phenylborsäure umsetzt (T.I. Perun, I.R. Martin und R.S. Egan, J. Org. Chem. 39, 1490 (1974) und dann selektiv die 15-Hydroxylgruppe acyliert.

Im Vergleich zu $PGD_2$-Derivaten zeichnen sich die neuen 11-Halogen-prostanglandine durch größere Stabilität aus.

Die neuen 11-Halogen-prostanderivate der allgemeinen Formel I sind wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Die erfindungsgemäßen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen. Sie wirken außerdem an der Leber und auch an der Bauchspeicheldrüse zytoprotektiv.

Einige der Verbindungen wirken blutdrucksenkend, regulierend bei Herzrhythmusstörungen und hemmend auf die Plättchenaggregation mit den sich daraus ergebenden Einsatzmöglichkeiten. Die neuen Prostaglandine können auch in Kombination zum Beispiel mit $\beta$-Blockern und Diuretika verwendet werden.

Die neuen Prostaglandin-Derivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen

usw. Ferner eignen sich die erfindungsgemäßen Prostaglandin-Derivate zur Cervixdilatation als Vorbereitung für diagnostische oder therapeutische Eingriffe.

Die gute Gewebsspezifität der erfindungsgemäßen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemäßen Substanzen wirken auch bronchospasmolytisch. Außerdem bewirken sie eine Abschwellung der Nasenschleimhaut.

Die neuen Prostaglandin-Analoga wirken stark luteolytisch, das heißt zur Auslösung einer Luteolyse benötigt man wesentlich geringere Dosierungen als bei den entsprechenden natürlichen Prostaglandinen.

Auch zur Auslösung von Aborten, insbesondere nach oraler oder intravaginaler Applikation, sind wesentlich geringere Mengen der neuen Prostaglandinanaloga im Vergleich zu den natürlichen Prostaglandinen erforderlich.

Bei der Registrierung der isotonischen Uteruskontraktion an der narkotisierten Ratte und am isolierten Rattenuterus zeigt sich, daß die erfindungsgemäßen Substanzen wesentlich wirksamer sind und ihre Wirkungen länger anhalten als bei den natürlichen Prostaglandinen.

Für die medizinische Anwendung können die Wirkstoffe in eine für die Inhalation, für orale, parenterale oder lokale (zum Beispiel vaginale) Applikation geeignete Form überführt werden.

Zur Inhalation werden zweckmäßigerweise Ärosollösungen hergestellt.

Für die orale Applikation sind beispielsweise Tabletten, Dragees oder Kapseln geeignet.

Für die parenterale Verabreichung werden sterile, injizierbare, wäßrige oder ölige Lösungen benutzt.

Für die vaginale Applikation sind zum Beispiel Zäpfchen geeignet und üblich.

Die Erfindung betrifft damit auch Arzneimittel auf Basis der Verbindungen der allgemeinen Formel I und der üblichen Hilfs- und Trägerstoffe.

Die erfindungsgemäßen Wirkstoffe sollen in Verbindung mit den in der Galenik bekannten und üblichen Hilfsstoffen, zum Beispiel zur Herstellung von Präparaten zur Auslösung eines Abortes, zur Zyklussteuerung, zur Einleitung einer Geburt oder zur Behandlung der Hypertonie dienen. Für diesen Zweck aber auch für die übrigen Anwendungen können die Präparate 0,01 - 50 mg der aktiven Verbindung enthalten.

Die folgenden Beispiele sollen die Erfindung näher erläutern, ohne daß damit eine Begrenzung vorgenommen wird.

## Beispiel 1

### (5Z,13E)-9α,15α-Dihydroxy-11β-chlor-16,16-dimethyl-5,13-prostadiensäure

a) 100 mg (0,25 mmol) (5Z,13E)-9α,11α,15α-Trihydroxy-16,16-dimethyl-prostadiensäure-methylester löste man in 20 ml absolutem Acetonitril-Pyridin (1:1), fügte 0,5 ml $CCl_4$ dazu und kühlte auf -8 °C. Dann wurde eine Lösung von 0,262 g (1 mmol) Triphenylphosphin in 10 ml Pyridin-Acetonitril (9:1) zugegeben und die Reaktionsmischung unter Rühren langsam auf 24 °C erwärmt und noch 16 Stunden bei 24 °C belassen. Nach Zugabe von 10 ml Toluol wurde abgedampft, der Rückstand in 30 ml $CH_2Cl_2$ gelöst und mit 25 ml gesättigter $NaHCO_3$-Lösung ausgeschüttelt und die wäßrige Lösung mit $CH_2Cl_2$ nachextrahiert. Nach Trocknen ($Na_2SO_4$) und Abdampfen löste man den Rückstand in 15 ml Toluol und chromatographierte an 100 g Silicagel, wobei durch Elution mit Toluol-Essigester (9:1) 54 mg (52 %) 9α,15α-Dihydroxy-11β-chlor-16,16-dimethyl-prostadiensäuremethylester erhalten wurden.

b) 54 mg des Methylesters wurden in 15 ml Methanol gelöst und mit 1 ml 0,5 N KOH-Lösung bei 0 °C versetzt und 72 Stunden bei 23 °C gerührt, eingedampft, mit $H_2O$ und fester Citronensäure versetzt und mit $CH_2Cl_2$ extrahiert, wobei 45,8 mg (88 %) hellbraunes Öl der Titelverbindung, die dünnschichtchromatographisch einheitlich war, erhalten wurden.

IR (Film): 615, 950, 1065, 1205, 1360, 1380, 1710, 2920, 2960/$cm^{-1}$

## Beispiel 2

### (5Z,13E)-9α,15α-Dihydroxy-11β-brom-16,16-dimethyl-5,13-prostadiensäure

100 mg (0,25 mmol) (5Z,13E)-9α,11α,15α-Trihydroxy-16,16-dimethyl-prostadiensäure-methylester setzte man analog zu Beispiel 1 in absolutem Acetonitril-Pyridin (1:1) mit Triphenylphosphin und $C_2Br_2Cl_4$ um, verseifte den Methylester und erhielt in ca. 30 % Gesamtausbeute die Titelverbindung.

## Beispiel 3

(5Z,13E)-9α,15α-Dihydroxy-11α-chlor-16,16-dimethyl-5,13-prostadiensäure

a) 2,5 g (5 mmol) (5Z,13E)-9α-Benzoyloxy-11α,15α-dihydroxy-16,16-dimethyl-5,13-prostadiensäuremethylester in 100 ml absolutem Toluol wurden bei 24 °C mit 6,58 g (25 mmol) Triphenylphosphin und 1,2 g (3 mmol) Zink-Tosylat versetzt und schließlich langsam während 25 Minuten 3,93 ml (25 mmol) Azodicarbonsäurediäthylester unter Rühren zugetropft. Darauf rührte man noch 1 Stunde bei 24 °C, setzte 100 ml $H_2O$ zu und extrahierte zweimal mit 400 ml Äther, wusch mit gesättigter NaCl-Lösung neutral und trocknete die Lösung mit $MgSO_4$. Nach Abdampfen wurde der Rückstand mit Hexan-Äther (4:1, dann 1:1) an einer Säule von 250 g Silicagel chromatographiert, wobei 1,733 g (53 %) an (5Z,13E)-9α-Benzoyloxy-11β-tosyloxy-15-hydroxy-16,16-dimethyl-prostadiensäuremethylester erhalten wurden.

b) Eine Lösung von 250 mg (0,38 mol) der obigen 11β-Tosyloxyverbindung wurde in 11,3 ml absolutem DMF mit 161 mg getrocknetem Lithiumchlorid 4 Stunden unter Argon auf 65 °C erhitzt, abgekühlt und mit 200 ml eiskalter gesättigter NaCl-Lösung versetzt. Nach Extraktion mit 400 ml Äther wurde getrocknet ($Na_2SO_4$) und eingedampft und der Rückstand an 100 g Silikagel chromatographiert. Elution mit Hexa-Äther (1:1) lieferte 139,3 mg (70,5 %) der gewünschten 11α-Chlorverbindung.

c) Zur Verseifung wurden 128,8 mg der vorstehend beschriebenen Verbindung mit überschüssigem KOH in 5 ml $H_2O/CH_3OH$ 3 Stunden bei 24 °C gerührt, abgedampft, mit wäßriger Citronensäure versetzt und mit Methylenchlorid extrahiert. Chromatographie an Kieselgel mit Hexan-Essigester ergab ca. 70 mg der Titelverbindung.

IR (Film): 840, 975, 1365, 1385, 1407, 1455, 1710, 2870, 2940, 2960 $cm^{-1}$

d) Der als Ausgangsmaterial für a) beschriebene (5Z,13E)-9α,15α-Dibenzoyloxy-11α-hydroxy-16,16-dimethyl-5,13-prostadiensäuremethylester wurde analog DOS 31 06 149 durch Horner-Wittig-Reaktion des (5E)-9α-Benzoyloxy-11α-tetrahydropyran-2-yloxy)-12β-formyl-13,14,15,16, 17,18,19,20nor-5-prostensäuremethylesters mit 2-(1,1-Dimethylpentyl)-2-oxoäthanphosphonsäuredimethylesters, $NaBH_4$-Reduktion, 15-Epimerentrennung und Entfernung der 11α-Tetrahydropyranyloxy-Schutzgruppe mit Essigsäure-$H_2O$-THF dargestellt.

Beispiel 4

(5Z,13E)-9α,15α-Dihydroxy-11α-brom-16,16-dimethyl-5,13-prostadiensäure

Analog zur Vorschrift 3 b) wurde durch Umsetzung von (5Z,13E)-9α-Benzoyloxy-11β-tosyloxy-15α-hydroxy-16,16-dimethylprostadiensäuremethylester mit wasserfreiem Lithiumbromid in absolutem DMF (5Z,13E)-9α-Benzoyloxy-11α-brom-15α-hydroxy-16,16-dimethyl-prostadiensäuremethylester dargestellt. Vorsichtige Verseifung mit 3 - 4 Äquivalenten Lithiumhydroxyd in Methanol-$H_2O$ (7 Stunden, 24 °C), Ansäuern mit Citronensäure und Extraktion mit $CH_2Cl_2$ ergab nach Chromatographie an Silicagel reines (5Z,13E)-9α,15α-Dihydroxy-11α-brom-16,16-dimethylprostadiensäure.

Beispiel 5

(5Z,13E)-9α,15α-Dihydroxy-11α-fluor-16,16-dimethyl-5,13-prostadiensäure

a) 1,34 g (2,05 mmol) (5Z,13E)-9α-Benzoyloxy-11β-tosyloxy-15α-hydroxy-16,16-dimethyl-5,13-prostadiensäuremethylester wurden in 75 ml Aceton gelöst und mit 7,36 ml (7,4 mmol) einer Lösung von absolutem Tetrabutylammoniumfluorid in THF versetzt und 96 Stunden bei 24 °C gerührt. Nach Aufarbeitung wurde mit Hexan-Äther (4:1, dann 1:1) an 150 g $SiO_2$ chromatographiert, wobei 154 mg (15 %) reiner (5Z,13E)-9α-Benzoyloxy-11α-fluor-15α-hydroxy-16,16-dimethyl-5,13-prostadiensäuremethylester isoliert wurden.

b) Verseifung des nach Beispiel 5a)hergestellten Methylesters mit KOH/Methanol/$H_2O$ (4 Stunden, 24 °C), Aufarbeitung und Chromatographie an $SiO_2$ mit Hexan-Essigester lieferte reine (5Z,13E)-9α,15α-Dihydroxy-11α-fluor-16,16-dimethyl-5,13-prostadiensäure.

Beispiel 6

(5Z,13E)-9α-Hydroxy-11β-chlor-15,15-ethylendioxy-16-phenoxy-17,18,19,20-nor-5,13-prostadiensäuremethylester

Zu einer Lösung von 0,8939 (2 mmol) (5Z,13E)-9α,11α-Dihydroxy-15,15-ethylendioxy-16-phenoxy-17,18,19,20-nor-5,13-prostadiensäuremethylester [vergl. W. Skuballa et al., J. Med. Chem. 21, 443 (1978)] und 0,97 ml (10 mmol) $CCl_4$ in 70 ml absolutem Acetonitril-Pyridin (1:1) wurde bei 24 °C unter Rühren eine Lösung von 1,31 g (5 mmol) Triphenylphosphin in 35 ml absolutem Acetonitril-Pyridin (1:1) während 2 Stunden zugetropft und über Nacht bei 24 °C gerührt. Nach Abdampfen unter Zusatz von Toluol wurde der Rückstand mit Toluol-Essigester an 60 g Silicagel chromatographiert, wobei 0,53 g (57 %) der Titelverbindung erhalten wurden.

IR (Film): 690, 755, 950, 980, 1050, 1080, 1174, 1440, 1455, 1495, 1590, 1600, 1735, 2890, 2950 $cm^{-1}$

Beispiel 7

(5Z,13E)-9α,15α-Dihydroxy-11β-chlor-16-phenoxy-17,18,19,20-nor-5,13-prostadiensäure

a) 400 mg (1 mmol) (5Z,13E)-9α-hydroxy-11β-chlor-15,15-ethylendioxy-16-phenoxy-17,18,19,20-nor-5,13-prostadiensäuremethylester rührte man mit 30 mg p-Toluolsulfonsäuremonohydrat in 100 ml Methanol 24 Stunden bei 24 °C, fügte nochmals 30 mg p-Toluolsulfonsäure-Hydrat hinzu und rührte wieder 24 Stunden bei 24 °C. Nach Abdampfen wurde der Rückstand mit $CH_2Cl_2$ und eiskalter $NaHCO_3$-Lösung geschüttelt, mit $CH_2Cl_2$ nachextrahiert und die $CH_2Cl_2$-Extrakte getrocknet ($Na_2SO_4$) und abgedampft, wobei 380 mg eines zähen Öls erhalten wurden.

Nach Lösen in 60 ml Methanol reduzierte man durch langsame Zugabe von 90 mg $NaBH_4$ bei 0 °C unter Rühren, neutralisierte schließlich mit wenig Essigsäure und dampfte ab. Nach Aufarbeitung mit $CH_2Cl_2$ und eiskalter $NaHCO_3$ -Lösung wurden 360 mg Rohprodukte erhalten. Chromatographie an 100 g $SiO_2$ mit Toluol-Essigester (9:1) lieferte 123 mg reines 15α-Epimeres, 89 mg 15-Epimerengemisch und schließlich 128 mg reines 15β-Epimeres.

b) Verseifung von 65 mg (0,15 mmol) des α-Epimeren mit 1 ml 0,5 N KOH in 20 ml Methanol (48 Stunden, 24 °C) ergab nach Abdampfen und Aufarbeitung mit Citronensäure/$CH_2Cl_2$ 35 mg (55,7 %) der Titelverbindung.

IR (Film): 695, 760, 975, 1040, 1030, 1175, 1245, 1460, 1500, 1710, 2870, 2930 $cm^{-1}$

Beispiel 8

(5Z,13E)-11β-Chlor-9α,15α-dihydroxy-16,19-dimethyl-5,13,18-prostatriensäure

342 mg (5Z,13E)-(8R,9S,11R,12R,15S,16RS)-9,15-Dibenzoyloxy-11-hydroxy-16,19-dimethyl-5,13,18-prostatriensäuremethylester wurden nach der in Beispiel 1 angegebenen Vorschrift mit 5,63 ml des dort beschriebenen Gemisches sowie mit 298 mg Triphenylphosphin in 3,6 ml Acetonitril und 0,5 ml Pyridin (Zutropfseit 1,5 Stunden) 19 Stunden bei Raumtemperatur umgesetzt. Nach säulenchromatographischer Reinigung an Kieselgel mit Hexan/Essigester (5:1) erhielt man 323 mg der 11β-Chlorverbindung.

IR (Film): 1737, 1720, 1601, 1584, 1491, 1272, 973, 712/cm.

Zur Verseifung wurden 294 mg der vorstehend beschriebenen 11β-Chlorverbindung mit 4,73 ml des in Beispiel 1 angegebenen Kaliumhydroxid/Wasser/Methanol-Gemisches 22,5 Stunden bei Raumtemperatur umgesetzt. Nach Reinigung durch Säulenchromatographie an Kieselgel mit Essigester als Fließmittel erhielt man 118 mg der Titelverbindung.

IR (Film): 3400 (breit), 2730, 2650, 1709, 973/cm.

Beispiel 9

(5Z,13E)-11β-Fluor-9α,15α-dihydroxy-16,19-dimethyl-5,13,18-prostatriensäuremethylester

Eine Losung von 430 mg (5Z,13E)-9α,15α-Dibenzoyloxy-11α-hydroxy-16,19-dimethyl-5,13,18-prostatriensäuremethylester in 0,7 ml Pyridin wurde bei 0 °C unter Argon mit 273 mg p-Toluolsulfonylchlorid versetzt und 13 Stunden nachgerührt. Anschließend wurde das Reaktionsgemisch mit 0,4 ml Wasser versetzt, 2 Stunden nachgerührt, mit Äther verdünnt, mit Wasser zweimal mit je 10 ml kalter 5 %iger Schwefelsäure, mit 10 ml Wasser, mit 10 ml gesättigter Natriumhydrogencarbonat-Lösung und nochmals mit Wasser gewaschen, über Magnesiumsulfat getrocknet und zur Trockne eingeengt. Man erhielt 514 mg des 11α-Tosylats, die ohne weitere Reinigung in die nächste Stufe eingesetzt wurden.

IR (Film): 1737, 1718, 1601, 1584, 1492, 1363, 1273, 1178, 970, 910, 713/cm.

503 mg des vorstehend beschriebenen 11α-Tosylats wurden in 23,9 ml Aceton gelöst und bei

Raumtemperatur unter Argon mit einer Lösung von 2,39 ml 1M Tetrabutylammoniumfluorid in 5 ml Tetrahydrofuran innerhalb von 1,75 Stunden tropfenweise versetzt. Man ließ 42 Stunden nachrühren, engte dann das Reakticnsgemisch am Rotationsverdampfer ein, versetzte mit 30 ml Wasser und extrahierte dreimal mit je 150 ml Methylenchlorid. Die vereinigten organischen Phasen wurden mit gesättigter Natriumchlorid-Lösung gewaschen, über Magnesiumsulfat getrocknet und zur Trockne eingeengt. Nach Säulenchromatographie an Kieselgel mit Hexan/25 - 33 % Äther als Fließmittel erhielt man 280 mg der gewünschten 11β-Fluorverbindung im Gemisch mit der entsprechenden $\Delta^{11,12}$-Verbindung, von denen 221 mg der Verseifung nach der Vorschrift in Beispiel 2 (20,5 mg Lithiumhydroxid, 4,8 ml Wasser/Methanol (1:2), 17,5 Stunden Reaktionszeit bei Raumtemperatur) unterworfen wurden. Da unter diesen Reaktionsbedingungen schon eine teilweise Methylester-Spaltung zu beobachten war, wurde eine Nachveresterung mit äthischer Diazomethan-Lösung notwendig. Nach Abtrennung des polareren Olefins durch Säulenchromatographie an Kieselgel mit Hexan/20 % Äther als Fließmittel erhielt man 10 mg der Titelverbindung.

IR (Film): 3400 (breit), 1722, 976/cm.

Beispiel 10

(5Z,13E)-11β-Chlor-9α,15α-dihydroxy-16,16,19-trimethyl-5,13,18-prostatriensäure

316 mg (5Z,13E)-9α,15α-Dibenzoyloxy-11α-hydroxy-16,16,19-trimethyl-5,13,18-prostatriensäuremethyiester wurden nach der in Beispiel 1 angegebenen Vorschrift mit 5,1 ml des dort beschriebenen Gemisches sowie mit 269 mg Triphenylphosphin in 3,3 ml Acetonitril und 0,36 ml Pyridin (Zutropfzeit 2 Stunden) 21 Stunden bei Raumtemperatur umgesetzt. Nach säulenchromatographischer Reinigung an Kieselgel mit Hexan/Essigester (5:1) erhielt man 320 mg der 11β-Chlorverbindung.

IR (Film): 1735, 1720, 1601, 1584, 1490, 1271, 975, 712/cm.

Zur Verseifung wurden 305 mg der vorstehend beschriebenen 11β-Chlorverbindung mit 9,6 ml des in Beispiel 1 angegebenen Kaliumhydroxid/Wasser/Methanol-Gemisches 28,5 Stunden bei Raumtemperatur umgesetzt. Nach Reinigung durch Säulenchromatographie an Kieselgel mit Hexan/50 - 100 % Essigester als Fließmittel erhielt man 91 mg der Titelverbindung. IR (Film): 3420 (breit), 2730, 2660, 1709, 976/cm.

## Patentansprüche

**1.** 11-Halogen-prostanderivate der allgemeinen Formel I

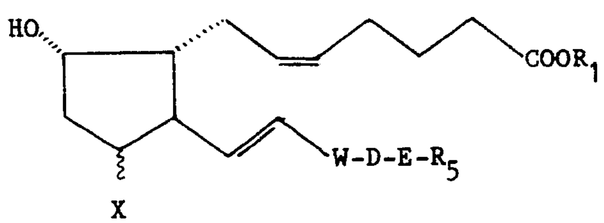

(I),

worin

X F, Cl oder Br,

$R_1$ Methyl wenn X, F oder Cl, D die Gruppen $-CH_2-$ oder $-CH(CH_3)-CH_2-$,E Sauerstoff oder eine direkte Bindung und $R_5$ Phenyl oder den Rest $CH=C(CH_3)_2$ bedeuten, oder Wasserstoff,

W eine Äthylendioxy-methylengruppe, wenn $R_1$ Methyl, X Cℓ, D die Gruppe $-CH_2-$, E Sauerstoff und $R_5$ Phenyl bedeuten, oder eine Hydroxymethylengruppe,

D die Gruppe $-CH_2-$, wenn E Sauerstoff und $R_5$ Phenyl bedeuten, oder D die Gruppe $-C(CH_3)_2-CH_2-$, wenn $ER_5$ n-Propyl oder den Rest $CH=C(CH_3)_2$ bedeuten, oder

$DER_5$ den Rest $-CH(CH_3)-CH_2-CH=C(CH_3)_2$,

$R_5$ $C_3$-$C_6$ Cycloalkyl, wenn D und E gemeinsam eine direkte Bindung bedeuten, oder Phenyl, wenn D eine $-CH_2$-Gruppe und E Sauerstoff bedeuten.

**2.** Verfahren zur Herstellung von 11-Halogen-prostanderivaten der allgemeinen Formel I, dadurch gekennzeichnet, daß man in an sich bekannter Weise eine Verbindung der allgemeinen Formel II

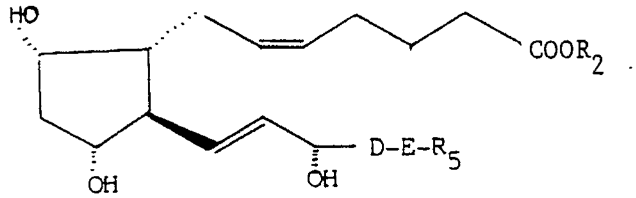

(II),

worin $R_2$ eine Alkylgruppe mit 1-4C-Atomen und D, E und $R_5$ die oben angegebenen Bedeutungen haben und freie OH-Gruppen in 9- und 15-Stellung gegebenenfalls geschützt sind

a) mit Triphenylphosphin/$CCl_4$ oder $C_2Cl_6$ direkt in die entsprechenden 11β-Chlorderivate überführt,
b) durch Reaktion mit p-Toluolsulfonylchlorid oder -anhydrid und anschließende Umsetzung mit einem Fluorid oder Chlorid zu den den entsprechenden 11β-Fluor- oder -Chlor-verbindungen umsetzt oder
c) mit Diäthylazodicarboxylat/Triphenylphosphin/Zink-tosylat in Tetrahydrofuran in die entsprechenden 11β-Tosyloxyderivate überführt und diese mit Fluorid- oder Chloridsalzen zu den entsprechenden 11α-Halogenprostansäurederivaten umsetzt und gegebenenfalls anschließend die vorhandenen Schutzgruppen abspaltet.

**3.** Arzneimittel, bestehend aus einer oder mehreren Verbindungen gemäß Anspruch 1 und üblichen Hilfs- und Trägerstoffen.

## Claims

**1.** 11-Haloprostane derivatives of the general formula I

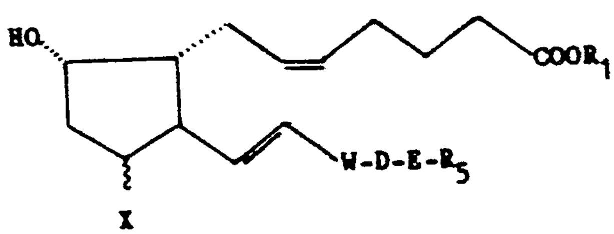

(I)

in which

| | |
|---|---|
| X | represents F, Cl or Br, |
| $R_1$ | represents methyl when X represents F or Cl, D represents the group $-CH_2-$ or $-CH(CH_3)-CH_2-$, E represents oxygen or a direct bond and $R_5$ represents phenyl or the radical $CH=C(CH_3)_2$, or $R_1$ represents hydrogen, |
| W | represents an ethylenedioxymethylene group when $R_1$ represents methyl, X represents Cl, D represents the group $-CH_2-$, E represents oxygen and $R_5$ represents phenyl, or W represents a hydroxymethylene group, |
| D | represents the group $-CH_2-$ when E represents oxygen and $R_5$ represents phenyl, or D represents the group $-C(CH_3)_2-CH_2-$ when $ER_5$ represents n-propyl or the radical $CH=C-(CH_3)_2$, |
| $DER_5$ | represents the radical $-CH(CH_3)-CH_2-CH=C(CH_3)_2$, |
| $R_5$ | represents $C_3$-$C_6$cycloalkyl when D and E together represent a direct bond, or represents phenyl when D represents a $-CH_2-$ group and E represents oxygen. |

**2.** A process for the preparation of 11-haloprostane derivatives of the general formula I, characterised in that, in a manner known per se, a compound of the general formula II

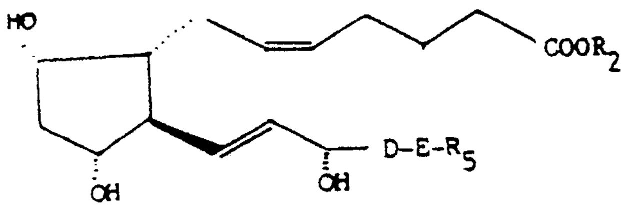

(II),

in which $R_2$ represents an alkyl group having from 1 to 4 carbon atoms, D, E and $R_5$ have the meanings given above and free OH groups in the 9- and 15-positions are optionally protected,

a) is converted with triphenylphosphine/$CCl_4$ or $C_2Cl_6$ directly into the corresponding 11β-chlorine derivative,
b) is converted by reaction with p-toluenesulphonyl chloride or anhydride and subsequent reaction with a fluoride or chloride to the corresponding 11β-fluorine or -chlorine compounds, or
c) is converted with diethylazodicarboxylate/triphenylphosphine/zinc tosylate in tetrahydrofuran into the corresponding 11β-tosyloxy derivative, and this is reacted with fluoride or chloride salts to form the corresponding 11α-haloprostanoic acid derivatives and optionally then the protecting groups present are removed,

3. Medicament, comprising one or more compounds according to claim 1 and customary excipients and carriers.

**Revendications**

1. Dérivés d'halogéno-11 prostanes répondant à la formule générale I :

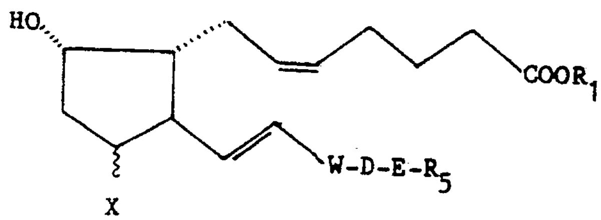

(I),

dans laquelle

| | |
|---|---|
| X | représente F, Cl ou Br, |
| $R_1$ | représente un méthyle lorsque X représente F ou Cl, D un radical $-CH_2-$ ou $-CH(CH_3)-CH_2-$, E l'oxygène ou une liaison directe et $R_5$ un phényle ou un radical $-CH=C(CH_3)_2$, ou représente l'hydrogène, |
| W | représente un radical éthylène-dioxy-méthylène lorsque $R_1$ représente un méthyle, X Cl, D un radical $-CH_2-$, E l'oxygène et $R_5$ un phényle, ou représente un radical hydroxyméthylène, |
| D | représente un radical $-CH_2-$ lorsque E représente l'oxygène et $R_5$ un phényle, ou D représente un radical $-C(CH_3)_2-CH_2$ lorsque $ER_5$ représente un radical n-propyle ou un radical $-CH=C(CH_3)_2$, |
| $DER_5$ | représente un radical $-CH(CH_3)-CH_2-CH=C(CH_3)_2$, |
| $R_5$ | représente un cycloalkyle en $C_3$-$C_6$ lorsque D et E forment ensemble une liaison directe, ou représente un phényle lorsque D est un radical $-CH_2-$ et E l'oxygène. |

2. Procédé pour préparer des dérivés d'halogéno-11 prostanes de formule générale I, procédé caractérisé en ce que, en partant d'un composé répondant à la formule générale II :

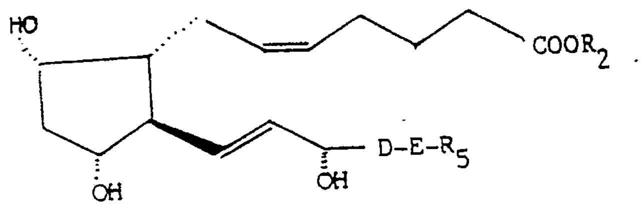

(II),

dans laquelle $R_2$ représente un radical alkyle contenant de 1 à 4 atomes de carbone, D, E et $R_5$ ont les significations qui leur ont été données ci-dessus, et des radicaux OH libres en position 9 et en position 15 sont éventuellement protégés, et en opérant de manière connue :

a) on transforme ce composé de formule II par réaction avec triphénylphosphine/$CCl_4$ ou $C_2Cl_6$, directement en le dérivé chloro-11$\beta$ correspondant,

b) on transforme ledit composé, par réaction avec le chlorure de p-toluène-sulfonyle ou l'anhydride p-toluène-sulfonique, puis réaction avec un fluorure ou un chlorure, en le composé fluoro-11$\beta$ ou chloro-11$\beta$ correspondant, ou

c) on transforme ledit composé, par réaction avec l'association azo-dicarboxylate de diéthyle/triphénylphosphine/tosylate de zinc, dans du tétrahydrofuranne, en le dérivé tosyloxy-11$\beta$ correspondant, on fait réagir ce dernier avec des sels de l'acide fluorhydrique ou de l'acide chlorhydrique pour obtenir le dérivé d'acide halogéno-11$\alpha$ prostanoïque correspondant, et ensuite, éventuellement, on coupe les radicaux protecteurs présents.

**3.** Médicament constitué d'un ou de plusieurs composés selon la revendication 1 et d'adjuvants et d'excipients usuels.